(19) Europäisches Patentamt
European Patent Office
Office européen des brevets

(11) Publication number: 0 123 071 B1

(12) # EUROPEAN PATENT SPECIFICATION

(45) Date of publication of patent specification: 31.01.90

(51) Int. Cl.$^5$: **A 61 K 7/06**, A 61 K 7/11

(21) Application number: 84102250.2

(22) Date of filing: 02.03.84

(54) **Hair setting compositions.**

(30) Priority: 22.04.83 JP 71153/83

(43) Date of publication of application:
31.10.84 Bulletin 84/44

(45) Publication of the grant of the patent:
31.01.90 Bulletin 90/05

(84) Designated Contracting States:
AT BE CH DE GB LI NL

(56) References cited:
DE-B-1 150 179
FR-A-2 101 044
FR-A-2 437 829

HUGO JANISTYN: "HANDBUCH DER KOSMETIKA UND RIECHSTOFFE", vol. 3, Edition 2, 1973, pages 288-289, Dr. Alfred Hüthig Verlag, Heidelberg, DE;

(73) Proprietor: Kao Corporation
14-10, Nihonbashi Kayabacho 1-chome
Chuo-Ku Tokyo 103 (JP)

(72) Inventor: Ando, Hiroshi
1-13-11, Koyadai Funabashi-shi
Chiba-ken (JP)
Inventor: Suzuki, Noboru
9-4-17, Kameido Koto-ku
Tokyo (JP)

(74) Representative: Wächtershäuser, Günter, Dr.
Tal 29
D-8000 München 2 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 123 071 B1

**Description**

i) Field of the Invention:

This invention relates to hair setting compositions which ensure excellent curl retentivity and agreeable feeling of the hair after setting.

ii) Description of the Prior Art:

Cold perms have been widely performed in order to have the hair waved or curled. After the cold perming, hair setting cosmetics such as setting lotions and hair sprays are used to set the hair therewith and to have the hair styled beautifully. The hair setting cosmetics may also be used to temporarily wave or curl non-permed hair or to prevent the hair from dishevelling.

Known hair setting compositions are usually prepared by dissolving hair setting polymer compounds (hereinafter referred to simply as "polymer compound") in a suitable solvent such as water, a lower alcohol, or a mixed solvent of water and a lower alcohol.

However, the thus obtained known hair setting compositions have the drawbacks that (1) when deposited on the hair, the compositions show stickiness during and after drying and thus spoil the texture of the hair considerably and (2) the hair applied with the composition cannot be readily combed or brushed and when combed, flaking takes place, resulting in a considerable lowering of set retentivity. In order to overcome the drawbacks, addition of oils and fats for cosmetics, silicone oils and surface active agents has been proposed without successful results.

Summary of the Invention

We have made studies on additives for hair setting compositions and found that addition of lecithin is effective in overcoming the drawbacks involved in the known hair setting compositions and that further addition of a plant extract leads to a more excellent hair setting effect.

According to the embodiment of the invention, there is also provided a hair setting composition which comprises (a) 0.02—10.0 wt% of a synthetic polymer compound ordinarily used for hair-setting purposes, (b) lecithin used in an amount of 0.1—2.0 times that of the ingredient (a) on the weight basis, and (c) up to 1.0 wt% of a plant extract.

Detailed Description of the Invention and Preferred Embodiments

The ingredient (a) of the invention may be any synthetic polymer compounds ordinarily used for this purpose. Natural polymer compounds such as guar are so sticky that the purposes of the invention cannot be achieved. Examples of the synthetic polymer compounds are indicated below.

(1) Polyvinylpyrrolidone Polymer Compounds

Typical of these compounds are polyvinylpyrrolidone, copolymers of vinylpyrrolidone and vinyl acetate, copolymers of vinylpyrrolidone, vinyl acetate and alkylaminoacrylates. These polymers are commercially available under the names of Luviskol K, Luviskol VA, Luviflex D4101 (Yuka Badische Co., Ltd.), PVPK, PVP/VA and E-735 (GAF Co., Ltd.).

(2) Acidic Vinyl Ether Polymer Compounds

Typical of the polymer compounds are lower alkyl half esters of copolymers of methyl vinyl ether and maleic anhydride, which are available under the names of Gantrez ES-225, ES-335 (GAF Co., Ltd.).

(3) Acidic Polyvinyl Acetate Polymer Compounds

Typical of these compounds are copolymers of vinyl acetate and crotonic acid and commercially sold products include Resin 28—1310 (National Starch Co., Ltd.) and Luviset CE5055 (Yuka Badische Co., Ltd.).

(4) Acidic Acrylic Polymer Compounds

Examples of these polymers include copolymers of acrylic and/or methacrylic acid and alkyl acrylates and/or methacrylates, copolymers of acrylic acid, alkyl acrylates and N-alkylacrylamides, copolymers of acrylic acid and polyhydric alcohols such as allyl sucrose (e.g. carboxyvinyl polymer). Commercial products of these polymers are Plascize (Gooh Chem Co., Ltd.), Ultrahold 8 (Ciba-Geigy A.G.) and Carbopol 941 (B. F. Goodrich Chemical Co., Ltd.).

(5) Amphoteric Acrylic Polymer Compounds

Examples of these polymers include those which are obtained by copolymerizing dialkylaminoethyl methacrylates, dialkylaminoethyl acrylates and diacetonacrylamides with acrylic acid, methacrylic acid, alkyl acrylates and alkyl methacrylates and subjecting the resulting copolymers to reaction with halogenated acetic acid to render them amphoteric. The polymers are available, for example, under the name of Yukaformer AM75 (Mitsubishi Petrochemical Co., Ltd.).

(6) Decomposition Derivatives of Keratin Materials

The derivatives are those which are obtained by oxidative decomposition of keratin materials or by

subjecting keratin material to reductive decomposition and chemically modifying the thiol group thereof. Examples of the keratin materials include animal hairs, human hair, feathers, nails, horns, hooves and scales of which wool and human hair and feathers are preferable.

The derivatives at the thiol group are as follows:

$-SCH_2COOH$, $-SCH_2CH_2COOH$, 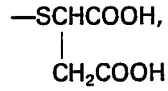,

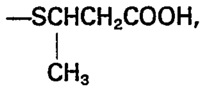, 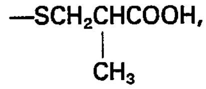,

$-SO_3H$, $-SSO_3H$, $-SCH_2CH_2SO_3H$,

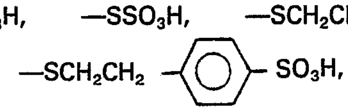,

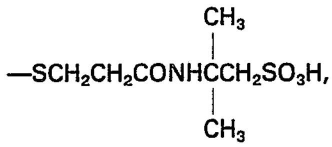, $-SCH_2CH_2SO_2CH_2COOH$.

Of these,

$-SCH_2COOH$ and 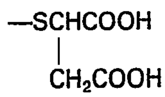

are preferred.

The chemical modification of the thiol group may be conducted by any known techniques as described, for example, in Textile Progress, Vol. 7, page 1 (1975), by N. H. Leon, "Organic Sulfur Compounds" by Shigeru Ooae (Published by Kagaku Dojin Pub. (1968)) and "Kobunshi Jikkengaku Koza", Vol. 12 by Masami Oku (Published by Kyoritsu Pub.).

Of the polymer compounds used as the ingredient (a), polymer compounds having an acidic group should preferably be neutralized with respect to part or a whole (50 to 100%) of the acidic groups in order to improve washability and feeling to the touch. The alkalis used for the neutralization are not limited to any specific ones. The acidic groups of polymer compounds may be converted into salts with alkali metals such as sodium and potassium, ammonium salts or salts with organic bases such as ethanolamine, diethanolamine, triethanolamine, 2-amino-2-methylpropanol, 2-amino-2-methyl-1,3-propanediol, aminomercaptopropanediol, triisopropanolamine, glycine, histidine and alginine.

The most preferable polymer compounds are acidic acrylic polymers of (4) including, for example, copolymers of diacetoneacrylamide, acrylic or methacrylic acid esters of aliphatic alcohols having from 4 to 18 carbon atoms, acrylic acid, methacrylic acid or itaconic acid with acrylic or methacrylic acid esters having from 1 to 3 carbon atoms (Japanese Patent Publication No. 50—6538), and copolymers of acrylic acid and allyl sucrose (Japanese Patent Publication No. 32—4141).

In the practice of the invention, one or more polymer compounds are added, as the ingredient (a), to the hair setting composition in an amount of from 0.02 to 10.0 wt% (hereinafter referred to simply as "%"), preferably from 0.05 to 3.0%.

The ingredient (b) used in the present invention is synthetic lecithin or natural lecithin which is a phospholipid contained in seeds of leguminous plants such as soybean, and yolk in large amounts. Such lecithin is commercially available as Nisshin Lecithin (from Nisshin Seiyu K.K.), Emulmetik 88 (from Lucasmeyer Co., Ltd.) and Yolk Lecithin (from Asahi Chem. Ind. Co., Ltd.). The ingredient (b) is added in an amount of from 0.1 to 2.0 times, preferably 0.5 to 1.5 times, the amount of the ingredient (a) on the weight basis.

The hair setting effect of the invention can be pronouncedly improved when a plant extract is used in combination.

The plant extract used in the present invention may be derived from a number of plants. Examples of the plants include birch, rosemary, arnica, hamamelis, camomile, sage, St. John's bread, henna, hop, lime, aloe, wild thyme, calendula, horsetail, mountain gentian, nettle, chestnut, avocado, seaweed, milfoil, coltsfoot, marigold, peach, rose, senna, thyme, white lily, tea, green tea. Of these plants, birch, rosemary, arnica, coltsfoot, hamamelis, camomile, sage, aloe, henna, St. John's bread, and tea are preferably used and most preferably, birch, rosemary, tea and hamamelis are used. Preferable sites or portions of the preferable plants to be extracted are, for example, bark of birch, the entirety of rosemary, leaves of hamamelis, flowers of camomile, leaves of sage, leaves of aloe, leaves of henna, seeds of St. John's bread, and leaves of tea.

The plant extract is obtained by subjecting flowers, leaves, seeds, roots and stems of the plants to solvent extraction according to any known procedure at a normal temperature or under heating conditions. The solvents for the extraction are polar solvents including, for example, lower alcohols such as methanol and ethanol, other organic solvents such as propylene glycol, 1,3-butylene glycol and glycerine and water. These solvents may be used singly or in combination.

The extracts of birch and rosemary which are indicated as the most preferable plants for the purposes are commercially available: birch extract products include Birch Extract (from Novarom Co., Ltd.), Boulene MCF787 (from Gattefossè Co., Ltd.), Birch Extract (from Virmin Co., Ltd.) and Extrapone Birch Special (from Dragoco Co., Ltd.), and rosemary extract products include Rosemary Extract (from Novarom Co., Ltd.), Romarin MCF772 (Gattefossè Co., Ltd.), Phytelene EG009 (Virmin Co., Ltd.), and Rosemary Extract (from Novak Co., Ltd.).

The plant extract is added in an amount not exceeding 1.0% and preferably in the range of from 0.0002 to 1.0%, most preferably from 0.001 to 0.2%, of the hair setting composition as a residue which is separated from the extraction solvent by distillation when the extract is in the form of a solution.

The plant extract may be added directly to hair setting compositions in the form of a solution, or may be added in the form of a concentrate obtained by distilling off the extraction solvent to a desired level or completely.

The hair setting composition of the invention is prepared by dissolving the effective ingredients in a solvent such as water, a lower alcohol or a mixed solvent of water and a lower alcohol according to any known procedure and, if necessary, mixing the solution with a jetting agent.

When a setting lotion is prepared, it is preferable to use, as a solvent, water or a mixed solvent of a monohydric alcohol having from 2 to 3 carbon atoms and water. With hair sprays, a monohydric alcohol having from 2 to 3 carbon atoms and particularly ethanol is preferably used. Jetting agents used in aerosol are chloroalkane compounds including chlorofluoroalkanes such as trichloromonofluoromethane, dichlorodifluoromethane and dichlorotetrafluoroethane, liquefied petroleum gases, dimethyl ether and mixtures thereof. The jetting agent should preferably be charged into an aerosol can so that an internal pressure in the can is in the range of 2.0 to 4.0 $Kg/m^2G$. With a hair setting composition in the form of a misty aerosol, the jetting agent is preferably contained in an amount of from 50 to 80% or more. With a hair setting composition provided as a foamy aerosol, its content should preferably be in the range of from 10 to 30%.

Aside from the effective essential ingredients, the hair setting composition of the invention may further comprise, within ranges of amounts not impeding the effect of the invention, fats and oils for cosmetics including: glycerides such as castor oil, cacao oil, mink oil, avocado oil and olive oil; waxes such as bees wax, whale wax, lanolin and carnauba wax; alcohols such as cetyl alcohol, oleyl alcohol, hexadecyl alcohol, lauryl alcohol, stearyl alcohol, isostearyl alcohol, 2-octyl dodecanol, propylene glycol, polypropylene glycol and glycerine; esters such as isopropyl myristate, hexyl laurate, cetyl lactate, propylene glycol monostearate, oleyl oleate, hexadecyl 2-ethylhexanoate and octyldodecyl myristate; and silicone derivatives such as dimethylpolysiloxane, methylphenylpolysiloxane, polyether-modified silicone oils, epoxy-modified silicone oils, amino-modified silicone oils and alkyl-modified silicone oils. Additionally, emulsifiers may be added in order to stably emulsify these oils. Moreover, perfumes or colorants may be added so as to increase the commercial value. Preservatives or antioxidants may also be added in order to prevent the composition from deteriorating as time passes.

The thus obtained hair setting composition of the present invention is excellent in curl retentivity and agreeable to the touch when applied to the hair. Especially, when the composition is used as a setting lotion or hair spray, its effects become remarkable.

The present invention is described by way of examples, in which the performance evaluation of hair setting compositions is carried out according to the following methods.

(1) Set Retentivity

A hair bundle having a length of 18 cm and a weight of 1.5 g was wetted with water and applied with 0.3 g of a hair setting composition, followed by winding the bundle about a rod and drying naturally. After the drying, the curled hair bundle was removed from the rod and suspended in an air-conditioned room (25°C, 98% R.H.) for 30 minutes. An elongation of the curled hair bundle was measured to determine the set retentivity. For the determination, a length of the hair immediately after the removal was taken as a set retentivity of 100% and an original length (18 cm) of the curl-free hair was taken as a set retentivity of 0%.

(2) Feeling to the touch, stickiness, ease in brushing

The hair bundle curled in the same manner as in (1) was applied with hair setting compositions and naturally dried. Thereafter, the hair bundle was organoleptically evaluated by a panel of 10 female experts. The evaluation was indicated by an average value of the experts.

| (Evaluation Point) | Content |
|---|---|
| +3 | Much better than a control |
| +2 | Better than a control |
| +1 | Slightly better than a control |
| 0 | Equal to a control |
| −1 | Slightly poorer than a control |
| −2 | Poorer than a control |
| −3 | Much poorer than a control |

Example 1

A setting lotion composition of the following formulation was prepared and was evaluated with respect to its set retentivity, ease in brushing, feeling to the touch and stickiness. The results are shown in Table 1.

Formulation:

| | |
|---|---|
| Acrylic acid/methacrylic ester copolymer*[1] | 0.5% |
| Lecithin*[2] | 0 or 0.5 |
| Polyoxyethylene (50) hardened castor oil | 0.2 |
| Perfume | 0.1 |
| Absolute ethanol | 30.0 |
| Water | balance |

*[1]: Plascize L53P (Gooh Chem. Co., Ltd.)
*[2]: Emulmetik 88 (Lucasmeyer Co., Ltd.)

TABLE 1

| | Amount of Lecithin (%) | Evaluation | | | |
|---|---|---|---|---|---|
| | | Set retentivity | Feeling to the touch | Stickiness | Ease in brushing |
| Inventive Composition | 0.5 | 68 | +2.6 | +2.6 | +2.5 |
| Control | 0 | 36 | — | — | — |

Example 2

A setting lotion of the following formulation was prepared and was used to evaluate its set retentivity, ease in brushing, feeling to the touch and stickiness. The results are shown in Table 2.

Formulation:

| | |
|---|---|
| Acrylic acid/allyl sucrose copolymer*[1] | 0.5% |
| Lecithin*[2] | (Table 2) |
| Birch extract*[3] | (Table 2) |
| Polyoxyethylene (20) oleyl ether | 0.2 |
| Perfume | 0.1 |
| Absolute ethanol | 20.0 |
| Water | balance |

*[1]: Carbopol 941 (B. F. Goodrich Chemical Co., Ltd.)
*[2]: Emulmetik 88 (Lucasmeyer Co., Ltd.)
*[3]: Novarom Co., Ltd.

TABLE 2

| Amount (%) | | Evaluation | | | |
|---|---|---|---|---|---|
| Lecithin | Birch extract | Set retentivity | Feeling to the touch | Stickiness | Ease in brushing |
| Composition of Invention | | | | | |
| 0.7 | 0.03 | 82 | +2.4 | +2.7 | +2.5 |
| 0.7 | 0.2 | 83 | +2.6 | +2.7 | +2.6 |
| Comparation | | | | | |
| 0.01 | 0 | 45 | +0.2 | 0 | +0.1 |
| 1.5 | 0 | 51 | +0.5 | −0.4 | +1.3 |
| Control | | | | | |
| 0 | 0 | 32 | — | — | — |

Example 3

A setting lotion having the following formulation was prepared for evaluation of its set retentivity, ease in brushing, feeling to the touch and stickiness. The results are shown in Table 3.

Formulation:

| | |
|---|---|
| Acrylic acid/methacrylic ester copolymer*[1] | 0.5% |
| Lecithin*[2] | 0.5 |
| Plant extract (Table 3) | 0 or 0.3 |
| Polyoxyethylene (50) hardened castor oil | 0.2 |
| Perfume | 0.1 |
| Absolute ethanol | 30.0 |
| Water | balance |

*[1]: Plascize L53P (Gooh Chem. Co., Ltd.)
*[2]: Emulmetik 88 (Lucasmeyer Co., Ltd.)

TABLE 3

| | Plant extract | Evaluation: Set retentivity | Feeling to the touch | Stickiness | Ease in brushing |
|---|---|---|---|---|---|
| Invention | Hamamelis | 82 | +0.8 | +1.0 | +0.6 |
| | Tea | 85 | +1.0 | +0.9 | +0.5 |
| Control | — | 68 | — | — | — |

**Claims**

1. A hair setting composition characterized by comprising:
(a) 0.02 to 10.0 wt% of a synthetic polymer compound ordinarily used for hair setting purposes,
(b) lecithin used in an amount of 0.1—2.0 times that of the ingredient (a) on the weight basis, and
(c) up to 1.0 wt% of a plant extract.

2. The hair setting composition according to Claim 1, wherein said synthetic polymer compound is a polyvinylpyrrolidone polymer compound, an acidic vinyl ether polymer compound, an acidic polyvinyl acetate polymer compound, an acidic acrylic compound, an amphoteric polymer compound or a decomposition derivative of keratin material.

3. The hair setting composition according to Claim 1, wherein said plant extract is derived from the plant selected from the group consisting of birch, rosemary, arnica, hamamelis, camomile, sage, St. John's bread, henna, hop, lime, aloe, wild thyme, calendula, horsetail, mountain gentian, nettle, chestnut, avocado, seaweed, milfoil, coltsfoot, marigold, peach, rose, senna, thyme, white lily, tea and green tea.

**Patentansprüche**

1. Haarfestigerzusammensetzung, dadurch gekennzeichnet, daß sie umfaßt:
(a) 0,02 bis 10,0 Gew.-% einer synthetischen Polymerverbindung, wie sie herkömmlicherweise für Haarfestigerzwecke verwendet wird,
(b) Lecithin, das in einer Menge von 0,1 bis 2,0 mal der Menge des Bestandteils (a) auf Gewichtsbasis verwendet wird, und
(c) bis zu 1,0 Gew.-% eines Pflanzenextrakts.

2. Haarfestigerzusammensetzung gemäß Anspruch 1, wobei die synthetische Polymerverbindung eine Polyvinylpyrrolidon-Polymerverbindung, eine saure Vinylätherpolymerverbindung, eine saure Polyvinylacetat-Polymerverbindung, eine saure acrylische Verbindung, eine amphotere Polymerverbindung oder ein Zersetzungsderivat von Keratinmaterial ist.

3. Haarfestigerzusammensetzung gemäß Anspruch 1, wobei der Pflanzenextrakt aus Pflanzen stammt, ausgewählt aus der Gruppe bestehend auf Birke, Rosmarin, Arnika, Hamamelis, Damomil, Salbei, Johannisbrot, Henna, Hopfen, Linde, Limone, Aloe, wilder Thymian, Kalendula, Schachtelhalm, Gebirgsenzian, Nessel, Roßkastanie, Avokado, Meerespflanzen, Schafgarbe, Huflattich, Ringelblume, Pfirsich, Rose, Senna, Thymian, weiße Lilie, Tee und grüner Tee.

**Revendications**

1. Mélange pour mise en plis de cheveux caractérisé en ce qu'il est composé de:

(a) 0,02 à 10,0% en poids d'un composé d'un polymère synthétique utilisé habituellement pour effectuer une mise en plis de cheveux,

(b) de la lécitine utilisée en une proportion de 0,1—2,0 fois celle de l'ingrédient (a) sur base de poids, et

(c) jusqu'à 1,0% en % poids d'un extrait végétal.

2. Mélange pour mise en plis de cheveux selon la revendication 1, caractérisé en ce que ledit composé de polymère synthétique est un composé d'un polymère polyvinylepyrrolidone, un composé d'un polymère d'éther et de vinyle acidique, un composé d'acidique acrylique, un composé d'un polymère amphotérique ou une décomposition dérivée de matière kératine.

3. Mélange pour mise en plis de cheveux selon la revendication 1, caractérisé en ce que ledit extrait végétal est dérivé de la plante sélectionnée du groupe comprenant le bouleau, le romarin, l'arnica, l'hamamélis, la camomille, la sauge, le caroubier, le henné, le houblon, le limon, l'aloès, le thym sauvage, la calendule, la prêle, la gentiane des montagnes, l'ortie, la châtaigne, l'avocatier, les algues marines, le millefeuille, le tussilage, le souci, la pêche, la rose, le séné, le thym, le lis blanc, le thé et le thé vert.